# EUROPÄISCHE PATENTSCHRIFT

(11) **EP 1 380 565 B1**
(45) Veröffentlichungstag und Bekanntmachung des Hinweises auf die Patenterteilung: **14.03.2007**
(21) Anmeldenummer: 03013587.5
(22) Anmeldetag: 14.06.2003
(51) Int. Cl.: C07C 67/39

(54) **Verfahren zur Herstellung von Dimethylterephthalat**
Process for the production of dimethylterephtalate
Procédé pour la préparation de diméthyltéréphtalate

(30) Priorität: 06.07.2002 DE 10230487
(43) Veröffentlichungstag der Anmeldung: 14.01.2004
(73) Patentinhaber: H&G Hegmanns Ingenieurgesellschaft mbH, 45881 Gelsenkirchen (DE)
(72) Erfinder:
(74) Vertreter: Albrecht, Rainer Harald

(56) Entgegenhaltungen:
- EP-A- 0 916 644
- DE-C- 19 752 722

## Beschreibung

Die Erfindung betrifft ein Verfahren zur Herstellung von Dimethylterephthalat (DMT), bei dem
ein p-Xylol und p-Toluylsäuremethylester (PTE) enthaltendes Gemisch in flüssiger Phase in Gegenwart eines Schwermetallkatalysators mit einem sauerstoffhaltigen Gas oxidiert wird,
das Oxidationsprodukt mit Methanol zu einem Rohester verestert wird,
DMT aus dem Rohester isoliert wird und
der Schwermetallkatalysator sowie Fraktionen, die PTE und Zwischen- sowie Nebenprodukte enthalten, in den Prozess zurückgeführt werden,
wobei die Oxidation in mindestens zwei Oxidationsstufen durchgeführt wird.

Bei dem eingangs beschriebenen Verfahren zur Herstellung von DMT, das in der Literatur als Katzschmann-Verfahren oder Witten-Verfahren bezeichnet wird (Ullmanns Enzyklopädie der technischen Chemie, 4. Auflage, Band 22, Verlag Chemie 1982, Seiten 530 und 531), werden p-Xylol und p-Toluylsäuremethylester (PTE) gemeinsam in einem Gemisch mit Sauerstoff enthaltenden Gasen in Gegenwart von halogenfreien Schwermetallkatalysatoren, in der Regel Co/Mn-Mischungen, oxidiert. PTE wird dem Reaktionsgemisch im Überschuss zugesetzt, da es als Transport- und/oder Lösemittel für feste hochschmelzende Oxidationsprodukte dient, und aus nachfolgenden Verfahrensstufen bzw. Reaktoren in die Oxidation zurückgeführt. In einer der Oxidation nachfolgenden Veresterungskolonne wird das Oxidationsprodukt mit praktisch allen Zwischen- und Nebenprodukten der Oxidation verestert. Der aus der Veresterungskolonne abgezogene Rohester enthält im Wesentlichen PTE, DMT und Isomere von DMT sowie eine Mehrzahl von veresterten Zwischen- und Nebenprodukten. Diese Zwischen- und Nebenprodukte enthalten zunächst Wertstoffe, die wieder zu DMT umgesetzt werden können und hierzu zur Ausbeuteerhöhung in den Prozess zurückgeführt werden. Hierbei handelt es sich insbesondere um aromatische Alkohole, wie Hydroxymethylbenzoesäuremethylester (HMBME), um aromatische Aldehyde, wie Terephthalaldehydsäuremethylester (TAE) oder um in der Veresterung nicht umgesetzte Säuren wie p-Toluylsäure (PTS) und Monomethylterephthalat (MMT). HMBME und TAE sind veresterte Oxidationsprodukte, die aus der Oxidation von p-Xylol bzw. PTE resultieren. Die Zwischen- und Nebenprodukte enthalten aber auch unerwünschte Stoffe, die insbesondere bei Anreicherung die Verfahrensstufen nachteilig beeinflussen und die Ausbeute beeinträchtigen können. Darunter fallen beispielsweise Benzoesäuremethylester (BME) sowie mehrkernige aromatische Substanzen wie Diphenyle und Triphenyle.

Die Oxidation wird in zwei oder mehr Oxidationsstufen durchgeführt, wobei in der ersten Oxidationsstufe eine möglichst niedrige Temperatur angestrebt wird. Mit dem in den Oxidator zurückgeführten PTE-Strom gelangen beachtliche Mengen an hochschmelzenden Substanzen, z. B. DMT, sowie Substanzen, die in der Oxidation zu hochschmelzenden Substanzen weiter reagieren, z. B. TAE und HMBME in die Oxidationsstufe. Wegen der hohen Nebenproduktanteile im PTE kann die Temperatur in der ersten Oxidationsstufe nicht so weit abgesenkt werden, wie dies im Hinblick auf Selektivität und Raum/Zeit-Ausbeute wünschenswert wäre. Das in die Oxidation eingebrachte Gemisch enthält als Nebenprodukt auch BME sowie das Vorprodukt Benzoesäure (BS) in Konzentrationen bis zu 6 Gew.-%. Einerseits hält man den Gehalt an niedrig schmelzendem BME für vorteilhaft, da es ein gutes Lösungsmittel für hoch schmelzende Inhaltsstoffe darstellt und einer Ablagerung dieser Verbindungen entgegenwirkt. Andererseits wirken BME und BS emulgierend bzw. wasserbindend und sind deshalb dafür verantwortlich, dass mehr Wasser in der flüssigen Phase gebunden ist als dies aufgrund des Dampf/Flüssigkeitsgleichgewichtes erforderlich wäre. Das Wasser kann besonders bei niedrigen Temperaturen z. B. in der Nähe von Kühlrohren mit allgegenwärtiger Essigsäure als eigene Phase vorliegen und beachtliche Katalysatormengen aus der organischen Phase extrahieren und unwirksam machen. Bisher bekannte Ausschleusemethoden können auch bei sorgfältiger Betriebsweise diese Situation nicht wesentlich ändern. Man hilft sich in der Praxis damit, dass in der Oxidation höhere Temperaturen eingestellt werden, um die Konzentration des gelösten Wassers auf einem genügend niedrigem Niveau zu halten.

Der Erfindung liegt die Aufgabe zugrunde, bei dem eingangs beschriebenen Verfahren die Selektivität der Oxidation zu verbessern und dadurch den Rohstoffverbrauch zu reduzieren.

Zur Lösung dieser Aufgabe lehrt die Erfindung, dass aus dem Rohester eine leicht siedende PTE-Fraktion abgetrennt wird, die p-Toluylsäuremethylester (PTE), Benzoesäuremethylester (BME) und leichter siedende Stoffe sowie weniger als 5 Gew.-% DMT und Stoffe, die einen ähnlichen Siedepunkt wie DMT aufweisen, enthält, dass die leicht siedende PTE-Fraktion in die erste Oxidationsstufe zurückgeführt wird und dass die restliche PTE-Menge zusammen mit Hochsiedern, Oxidations- und Nebenprodukten mit einem ähnlichen Siedepunkt wie DMT und mit Wertprodukten, die bei der Aufarbeitung einer hochsiedenden Rohesterrückstandsfraktion erhalten werden, in einer nachfolgenden Oxidationsstufe eingesetzt werden. Gemäß einer bevorzugten Ausführung der Erfindung wird BME aus der niedrig siedenden PTE-Fraktion destillativ abgetrennt, bevor die PTE-Fraktion in die erste Oxidationsstufe zurückgeführt wird.

Das erfindungsgemäße Verfahren wird zweckmäßig so geführt, dass der PTE-Stoffmengenstrom, der mit der leicht siedenden PTE-Fraktion in der ersten Oxidationsstufe eingesetzt wird, mindestens 25 % der zugeführten p-Xylolmenge entspricht und dass der Gehalt an Benzoesäuremethylester (BME) im Rohester nach Abtrennung der leicht siedenden PTE-Fraktion weniger als 2,5 Gew.-% beträgt.

Die Oxidationsstufen werden mit unterschiedlichen Temperaturen betrieben, wobei in der ersten Oxidationsstufe eine möglichst niedrige Temperatur angestrebt wird. Mit dem erfindungsgemäßen Verfahren gelingt es, die Temperatur der ersten Oxidationsstufe auf unter 140 °C und vorzugsweise auf unter 135 °C abzusenken und die Selektivität wesentlich zu erhöhen, ohne dabei die Raum/Zeitausbeute zu verschlechtern und den Betrieb durch Ablagerungen oder Verstopfungen zu stören. Bei den erfindungsgemäß möglichen niedrigen Temperaturen kann die gesamte PTE-Menge bereits auf die erste Oxidationsstufe aufgegeben werden, da bei Temperaturen um 135 °C keine PTE-Oxidation stattfindet und deshalb schädlichen Nebenwirkungen niedriger Sauerstoffüberschüsse, wie sie bei einer überwiegenden p-Xylol-Oxidation im ersten Oxidator vorliegen, nicht ins Gewicht fallen. In der ersten Oxidationsstufe können bereits so große p-Xylol-Mengen oxidiert werden, dass die p-Xylol-Konzentrationen in einer nachfolgenden Oxidationsstufe so niedrig sind, dass für eine verlustarme PTE-Oxidation geeignete höhere Sauerstoffüberschüsse problemlos eingestellt werden können. Bei einer vorzugsweise dreistufigen Oxidation können auch im zweiten Oxidator relativ niedrige Temperaturen eingestellt werden. Der BME-Spiegel in der Oxidation kann mit dem erfindungsgemäßen Verfahren auf unter 1 Gew.-% reduziert werden, da auch die mit dem Rückxylol zurückgeführten Mengen wegen der niedrigeren Temperaturen erheblich reduziert werden.

Die leicht siedende PTE-Fraktion wird vorzugsweise durch ein- oder mehrstufige Entspannungsdestillation des Rohesters gewonnen, wobei die Entspannungsdestillation durch Strippung mit Methanol unterstützt werden kann. Wegen der guten Verstärkungswerte sind nur geringe Rückläufe erforderlich. Die leicht siedende PTE-Fraktion kann als Dephlegmat abgezogen und der methanolische Brüden als Reaktionsmedium im Prozess verwendet werden.

Wegen des guten Stoffübergangs bei der Rohesterstrippung ist eine Absenkung der BME-Konzentration im verbleibenden Rohester auf die erfindungsgemäßen Werte von vorzugsweise weniger als 2,5 Gew.-% unproblematisch. Die gesamte im Rohester enthaltene BME-Menge wird dabei bis auf geringe Reste in der niedrig siedenden PTE-Fraktion erhalten und kann aus dieser Fraktion destillativ abgetrennt werden. Dabei erhält man den gesamten in der Oxidation und der Veresterung gebildeten BME-Anteil. Von großem Vorteil ist ferner, dass auch die BME-Mengen mit abgeschieden werden, die aus der im Oxidat enthaltenen frisch veresterten Benzoesäure entstehen. Dies wäre bei einer Abscheidung aus dem Brüden der Veresterung nur zum geringen Teil und bei einer Abscheidung aus Stripperbrüden überhaupt nicht möglich, da die Reaktion der Benzoesäure noch nicht abgeschlossen ist bzw. noch gar nicht stattgefunden hat.

Bei dem erfindungsgemäßen Verfahren kann der Restsäuregehalt im Rohester gesteuert bzw. darin enthaltene Säuren mit Strippmethanol weiter verestert werden. Als regelndes Element dient der Wassergehalt des Strippmethanols. Vorteilhafterweise bleibt bei der Durchführung des erfindungsgemäßen Verfahrens vorwiegend Monomethylterephtalat im Rohester zurück, während p-Toluylsäure, die sich destillativ nicht von DMT trennen lässt, größtenteils verestert und als PTE abgestrippt wird. Gewisse Restsäurezahlen in Form von Monomethylterephtalat im Rohester sind vorteilhaft, da sie zur Lösung der Katalysatorkomponenten beitragen, die anderenfalls leicht zu Metall reduziert werden und als metallische Ablagerungen äußerst schwierig zu entfernende Ablagerungen in Apparaten und Rohrleitungen bilden. Bei dem erfindungsgemäßen Verfahren werden deshalb in der vor allen Dingen gefährdeten Veresterungskolonne mit Absicht erhöhte Restsäurezahlen zugelassen und erst in der Strippdestillation abgebaut. Da die Wirksamkeit des Katalysators bei dem erfindungsgemäßen Verfahren nicht beeinträchtigt wird, sind in der Oxidation nur Katalysatorkonzentrationen von 150 bis maximal 180 ppm erforderlich, so dass eine im Stand der Technik praktizierte Zugabe von Lithium zum Katalysator entfallen und die Verhinderung metallischer Ablagerungen nur durch Steuerung der Restsäurezahl im Rohester erfolgen kann.

In weiterer Ausgestaltung lehrt die Erfindung, dass PTE-Dephlegmat, welches aus dem Brüdenstrom einer Trennstufe gewonnen wird, und/oder ein PTE- und BME-haltiges Produkt, welches aus dem Brüden der Veresterungskolonne abgeschieden wird, der niedrig siedenden Fraktion hinzugefügt wird.

## Patentansprüche

1. Verfahren zur Herstellung von Dimethylterephthalat (DMT), bei dem
ein p-Xylol und p-Toluylsäuremethylester (PTE) enthaltendes Gemisch in flüssiger Phase in Gegenwart eines Schwermetallkatalysators mit einem sauerstoffhaltigen Gas oxidiert wird,
das Oxidationsprodukt mit Methanol zu einem Rohester verestert wird,
DMT aus dem Rohester isoliert wird und
der Schwermetallkatalysator sowie Fraktionen, die PTE und Zwischen- sowie Nebenprodukte enthalten, in den Prozess zurückgeführt werden,
wobei die Oxidation in mindestens zwei Oxidationsstufen durchgeführt wird, **dadurch gekennzeichnet, dass** aus dem Rohester eine leicht siedende PTE-Fraktion abgetrennt wird, die PTE, Benzoesäuremethylester (BME) und leichter siedende Stoffe sowie weniger als 5 Gew.-% DMT und Stoffe, die einen ähnlichen Siedepunkt wie DMT aufweisen, enthält, dass die leicht siedende PTE-Fraktion in die erste Oxidationsstufe zurückgeführt wird und dass die restliche PTE-Menge zusammen mit Hochsiedern, Oxidations- und Nebenprodukten mit einem ähnlichen Siedepunkt wie DMT und mit Wertprodukten, die bei der Aufarbeitung einer hochsiedenden Rohesterrückstandsfraktion erhalten werden, in einer nachfolgenden Oxidationsstufe eingesetzt werden.

2. Verfahren nach Anspruch 1, **dadurch gekennzeichnet, dass** der PTE-Stoffmengenstrom, der mit der leicht siedenden PTE-Fraktion in der ersten Oxidationsstufe eingesetzt wird, mindestens 25 % der zugeführten p-Xylolmenge entspricht und dass der Gehalt an Benzoesäuremethylester (BME) im Rohester nach Abtrennung der leicht siedenden PTE-Fraktion weniger als 2,5 Gew.-% beträgt.

3. Verfahren nach Anspruch 1 oder 2, **dadurch gekennzeichnet, dass** die leicht siedende PTE-Fraktion durch ein- oder mehrstufige Entspannungsdestillation des Rohesters gewonnen wird.

4. Verfahren nach einem der Ansprüche 1 bis 3, **dadurch gekennzeichnet, dass** die leicht siedende PTE-Fraktion bei einer Entspannungsdestillation des Rohesters durch Strippung mit Methanol gewonnen wird, wobei die leicht siedende PTE-Fraktion als Dephlegmat abgezogen wird und der methanolische Brüden als Reaktionsmedium im Prozess verwendet wird.

5. Verfahren nach einem der Ansprüche 1 bis 4, **dadurch gekennzeichnet, dass** der niedrig siedenden Fraktion PTE-Dephlegmat, das aus dem Brüdenstrom einer Trennstufe gewonnen wird, und/oder ein PTE- und BME-haltiges Produkt, welches aus dem Brüden der Veresterungskolonne abgeschieden wird, hinzugefügt wird.

6. Verfahren nach einem der Ansprüche 1 bis 5, **dadurch gekennzeichnet, dass** Benzoesäuremethylester (BME) aus der niedrig siedenden PTE-Fraktion destillativ abgetrennt wird.

7. Verfahren nach einem der Ansprüche 1 bis 6, **dadurch gekennzeichnet, dass** die Oxidationsstufen mit unterschiedlichen Temperaturen betrieben werden, wobei in der ersten Oxidationsstufe eine Temperatur von weniger als 140 °C eingestellt wird.

## Claims

1. A method of manufacturing dimethyl terephthalate (DMT), wherein
a mixture containing p-xylene and methyl p-toluate (PTE)is oxidised in the liquid phase in the presence of a heavy metal catalyst with an oxygen-containing gas,
the oxidation product is esterified with methanol to form a crude ester,
DMT is isolated from the crude ester and
the heavy metal catalyst and fractions containing PTE and intermediate products or by-products are recycled back to the process,
wherein the oxidation is conducted in at least two oxidation stages, **characterised in that** a low-boiling PTE fraction is separated from the crude ester, which contains PTE, methyl benzoate (BME) and lower-boiling components with less than 5 % by weight DMT and components with a similar boiling point to DMT, that the low-boiling PTE fraction is recycled to the first oxidation stage and that the remaining PTE along with high-boiling components, oxidation products and by-products with a similar boiling point to DMT and with valuable products, which are obtained during the processing of a high-boiling crude ester residual fraction, are used in a subsequent oxidation stage.

2. The method according to claim 1, **characterised in that** the flow of PTE material, which is used with the low-boiling PTE fraction in the first oxidation stage, corresponds to at least 25% of the p-xylene added and that the methyl benzoate (BME) content of the crude ester is less than 2.5% by weight following removal of the low-boiling PTE fraction.

3. The method according to claim 1 or 2, **characterised in that** the low-boiling PTE fraction is obtained by single or multi-stage vacuum distillation of the crude ester.

4. The method according to one of the claims 1 to 3, **characterised in that** the low-boiling PTE fraction is obtained through vacuum distillation of the crude ester by methanol stripping, wherein the low-boiling PTE fraction is drawn off as dephlegmated material and the methanol vapours are used as a reaction medium in the process.

5. The method according to one of the claims 1 to 4, **characterised in that** the PTE dephlegmated material, which is obtained from the vapour flow of a buffer stage, and/or a PTE and BME-containing product, which is separated from the esterification column vapours, is added to the low-boiling fraction.

6. The method according to one of the claims 1 to 5, **characterised in that** methyl benzoate (BME) is removed from the low-boiling PTE fraction by distillation.

7. The method according to one of the claims 1 to 6, **characterised in that** the oxidation stages are operated at different temperatures, wherein at the first oxidation stage a temperature of under 140 °C is set.

## Revendications

1. Procédé de préparation de diméthyltéréphtalate (DMT), dans lequel
un mélange contenant du p-xylol et du p-méthylester d'acide toluylique (PTE) est oxydé en phase liquide en présence d'un catalyseur de métal lourd avec un gaz contenant de l'oxygène ;
le produit d'oxydation est estérifié en ester brut à l'aide de méthanol ;
le DMT est isolé de l'ester brut et
le catalyseur de métal lourd ainsi que des fractions contenant du PTE et des produits intermédiaires ainsi que des sous-produits sont remis dans le processus,
l'oxydation étant réalisée en au moins deux étapes d'oxydation, **caractérisé en ce qu'**on isole de l'ester brut une fraction de PTE bouillant facilement qui contient du PTE, du méthylester d'acide benzoïque (BME) et des substances bouillant plus facilement ainsi que moins de 5 % en poids et des substances qui ont un point d'ébullition identique au DMT, que la fraction de PTE bouillant facilement est ramenée à la première étape d'oxydation et que la quantité résiduelle de PTE est mise en oeuvre avec des produits à haute ébullition, des produits d'oxydation et des sous-produits avec un point d'ébullition identique à celui du DMT et avec des produits valorisables qui sont obtenus lors de l'élaboration d'une fraction résiduelle d'ester brut à haute ébullition dans une étape d'oxydation suivante.

2. Procédé selon la revendication 1, **caractérisé en ce que** le flux quantitatif de PTE qui est mis en oeuvre avec la fraction de PTE bouillant facilement à la première étape d'oxydation équivaut à au moins 25 % de la quantité de p-xylol apportée et que la teneur en méthylester d'acide benzoïque (BME) de l'ester brut s'élève à moins de 2,5 % en poids après isolation de la fraction de PTE bouillant facilement.

3. Procédé selon la revendication 1 ou 2, **caractérisé en ce que** la fraction de PTE bouillant facilement est obtenue par distillation à détente en une ou plusieurs étapes de l'ester brut.

4. Procédé selon une des revendications 1 à 3, **caractérisé en ce que** la fraction de PTE bouillant facilement est obtenue par distillation à détente de l'ester brut par dépouillage au méthanol, la fraction de PTE bouillant facilement étant extraite sous forme de déphlegmat et que la vapeur de méthanol étant employée comme milieu de réaction dans le processus.

5. Procédé selon une des revendications 1 à 4, **caractérisé en ce qu'**à la fraction à basse ébullition, on ajoute du déphlegmat de PTE qui est obtenu à partir du flux de vapeur d'une étape d'isolation et/ou un produit contenant du PTE et du BME qui est isolé de la vapeur de la colonne d'estérification.

6. Procédé selon une des revendications 1 à 5, **caractérisé en ce que** du méthylester d'acide benzoïque (BME) est isolé par distillation de la fraction de PTE bouillant facilement.

7. Procédé selon une des revendications 1 à 6, **caractérisé en ce que** les étapes d'oxydation sont effectuées à des températures différentes, une température inférieure à 140° C étant établie dans la première étape d'oxydation.
